# EUROPÄISCHE PATENTSCHRIFT

(11) **EP 3 034 099 B1**
(45) Veröffentlichungstag und Bekanntmachung des Hinweises auf die Patenterteilung: **28.06.2017**
(21) Anmeldenummer: 14199005.1
(22) Anmeldetag: 18.12.2014
(51) Int. Cl.: A61L 9/00, A61L 2/18, A61L 2/20, A61L 2/22, B05B 17/06

(54) **Verfahren und System zur Entkeimung von Räumen aufweisend Wasserperoxid und ein Tensid**
Room sterilization method and system with aerosolized disinfectant comprising hydrogen peroxide and a surfactant
Méthode et système de sterilisation d'habitations avec du peroxyde d'hydrogène et un tensioactif

(43) Veröffentlichungstag der Anmeldung: 22.06.2016
(73) Patentinhaber: Klein, Klaus, 85055 Ingolstadt (DE)
(72) Erfinder: Klein, Klaus, 85055 Ingolstadt (DE)
(74) Vertreter: Stenger Watzke Ring

(56) Entgegenhaltungen:
- EP-A1- 2 759 309
- WO-A1-2009/037231
- WO-A1-2012/041910
- WO-A1-2014/076013
- WO-A1-2014/094019
- US-A1- 2005 152 991
- US-A1- 2011 085 934
- US-B2- 8 142 715

## Beschreibung

Die Erfindung betrifft ein Verfahren zur Entkeimung von Räumen.

Seit der Entdeckung der Übertragbarkeit von Krankheiten durch Keime im 19. Jahrhundert nimmt die Bedeutung der Hygiene im Allgemeinen und der Keimbekämpfung im Speziellen stetig zu. Insbesondere in hygienisch besonders sensiblen Umfeldern, wie zum Beispiel Krankenhäusern und Arztpraxen, aber auch in der Lebensmittelindustrie ist eine wirksame Keimbekämpfung von essentieller Wichtigkeit. Die Raumentkeimung hat sich diesbezüglich in der Praxis als besonders effektiv erwiesen.

Aus dem Stand der Technik sind zum Beispiel Verfahren bekannt, bei denen eine Raumentkeimung dadurch durchgeführt wird, dass der zu entkeimende Raum verschlossen, insbesondere versiegelt, und anschließend begast wird. Nach einer vorgebbaren Einwirkzeit von zum Beispiel zwei Stunden gilt der Raum als entkeimt. Aufgrund der Aggressivität des verwendeten Desinfektionsmittels, welches zumeist vergleichsweise hohe Konzentrationen an chlorhaltigen Verbindungen oder Ozon aufweist, dürfen sich Menschen während der Einwirkzeit nicht in diesem Raum aufhalten. Nach Abschluss der Entkeimung ist der Raum durchzulüften, um das eingesetzte Gas wieder zu entfernen. Alsdann kann der Raum wieder von Menschen betreten werden.

Nachteilig bei diesem Vorgehen ist, dass mit dem Lüften sowie mit dem Eintritt der Menschen in den Raum eine Wiederverkeimung stattfindet. Diese Wiederverkeimung geht derart schnell vonstatten, dass binnen kürzester Zeit der Keimstatus wiederhergestellt ist, wie er vor der Entkeimung bestand.

Zur Entkeimung von Luft ist es ferner bekannt, die zu entkeimende Luft mit elektromagnetischer Strahlung im ultravioletten Bereich des Spektrums zu bestrahlen. Hierzu wird Luft von einer entsprechenden Vorrichtung angesaugt, bestrahlt und wieder in den Raum eingeleitet. Eine Entkeimung der im Raum befindlichen Oberflächen, von insbesondere Wänden, Möbeln und dergleichen findet nicht statt, so dass eine Wiederverkeimung durch Kontakt der Luft mit besagten Oberflächen nicht verhindert werden kann.

Insbesondere zur Oberflächenentkeimung ist es aus dem Stand der Technik ferner bekannt, diese lokal mit einem Desinfektionsmittel zu besprühen und händisch, insbesondere durch wischen, zu reinigen. Es kommen zu diesem Zweck Tücher oder ähnliche Reinigungshilfen zum Einsatz, die mit besagtem Desinfektionsmittel besprüht werden müssen, oder bereits vorbehandelt sind. Es folgt ein Abwischen der zu reinigenden Oberflächen. Von Nachteil auch dieses Verfahrens ist, dass eine Wiederverkeimung stattfindet. Darüber hinaus ist die Qualität der Entkeimung von der Sorgfältigkeit beim Wischen abhängig. Hinzukommt, dass mit Hilfe des Wischens nicht alle Oberflächen gereinigt werden, sei es, dass diese nur schwer zugänglich sind oder dass sie überhaupt nicht abgewischt werden können, wie zum Beispiel aus Stoff gefertigte Gardinen, Bettwäsche, Tischdecken oder dergleichen.

Es ist ferner aus der DE 10 2010 020 508 A1 eine Injektionsvorrichtung bekannt, die zur Einleitung von Desinfektionsmitteln und/oder Duftstoffen in einen Volumenstrom eines Leitungssystems geeignet ist. Hierbei werden die Mittel, vorzugsweise in flüssiger Form, in das Leitungssystems eingedüst. Hierzu ist ein Injektionskopf vorgesehen, der insbesondere als Düse und besonders bevorzugt als Hohlkegeldüse ausgebildet sein kann. Die Eindüsung erfolgt hierbei intervallweise. Nachteilig hieran ist, dass in den Intervallpausen, in denen keinerlei Begasung stattfindet, eine Wiederverkeimung auftritt. Darüber hinaus hat sich herausgestellt, dass die sich aus der Kombination von Injektionsvorrichtung und Desinfektionsmittel ergebende Desinfektionsleistung insbesondere aufgrund der mangelnden Verteilbarkeit im Raum keine ausreichende Desinfektion gewährleisten kann.

In diesem Zusammenhang beschreibt die WO 2009/037231 ein Verfahren zur Desinfektion von Räumen mittels eines antimikrobiellen Aerosols. Das Aerosol ist dabei aus Wasserstoffperoxid, einer zyklischen Carbonsäure oder einem Salz hiervon und einem Feuchthaltemittel, wie insbesondere Glycerol gebildet. Das Aerosol wird vorzugsweise mit einer Ultraschallmembran erzeugt, wobei die Wasserstoffperoxidkonzentration im Aerosol zwischen 25 und 100 ppm liegt.

Die US 2005/0152991 A1 offenbart ein antimikrobielles Desinfektionsmittel aufweisend Peroxycarbonsäuren mittlerer Kettenlänge und ein Tensid. Besagtes Desinfektionsmittel kann zur Desinfektion von Objekten und Oberflächen eingesetzt werden. Zu diesem Zweck kann es als Aerosol appliziert werden.

Aus der US 8,142,715 B2 ist ein Verfahren zur Deaktivierung biologischer Stoffe auf Oberflächen mittels Aerosolbesprühung und nachfolgender Behandlung mit ultraviolettem Licht bekannt. Hierbei wird ein Fotosensibilisator auf die zu desinfizierende Oberfläche aufgebrüht. Dieser kann insbesondere aus Wasserstoffperoxid und/oder Peroxycarbonsäuren gebildet sein. Weitere Inhaltsstoffe des Fotosensibilisators können insbesondere Tenside sein. Die Desinfektionswirkung wird dadurch erreicht, dass die Peroxide während der UV-Behandlung in chemisch besonders aggressive Radikale zerfallen. Die Tröpchengröße im Aerosol liegt bei dieser Art der Behandlung vorzugsweise oberhalb von 50 µm.

Nachteilig an vorgenanntem Stand der Technik ist insbesondere, dass auch diese Methoden entweder gesundheitlich nicht vollständig unbedenklich oder hinsichtlich der Desinfektionswirkung verbesserungsbedürftig sind.

Es ist daher die **Aufgabe** der Erfindung, ein Verfahren zur Entkeimung von Räumen bereitzustellen, mit welchem eine gesundheitlich unbedenkliche Entkeimung erreicht und eine Wiederverkeimung verhindert wird.

Zur **Lösung** dieser Aufgabe wird ein Verfahren zur Entkeimung von Räumen oder Bereichen hiervon gemäß Anspruch 1 vorgeschlagen.

Durch die erfindungsgemäße kontinuierliche Verfahrensführung wird eine dauerhaft sichere Entkeimung erreicht und eine Wiederverkeimung wirksam vermieden. Kontinuierlich im Sinne der Erfindung bezeichnet hierbei die zeitlich beliebig lange Durchführung des Verfahrens ohne dass eine bestimmungsgemäße Nutzung des zu entkeimenden Raumes oder des Bereiches hiervon unterbrochen werden müsste.

Erfindungsgemäß wird hiermit eine kontinuierliche Abtötung von krankheitserregenden, gesundheitsschädlichen oder ähnlichen Keimen erreicht und eine Wiederverkeimung für die Dauer des angewendeten Verfahrens verhindert. Zu besagten Keimen zählen insbesondere Bakterien, Viren, Pilze, Sporen, Feinstäube und ähnliches.

Eine erfolgreiche kontinuierliche Verfahrensführung zur Entkeimung hängt erfindungsgemäß vom synergetischen Zusammenwirken einzelner erfindungswesentlicher Merkmale ab. So ist es zum einen erforderlich, die Einzelkonzentrationen der verwendeten Komponenten des Desinfektionsmittels unterhalb eines jeweils vorgebbaren Grenzwertes zu halten. Hierdurch wird eine Verfahrensführung ohne Beeinträchtigung der bestimmungsgemäßen Nutzung des zu entkeimenden Raumes ermöglicht. Das erfindungsgemäße Verfahren ist in einer Vielzahl von Bereichen Einsetzbar. Insbesondere im Gesundheitssektor, dort bevorzugt in Krankenhäusern, Pflegeheimen, Arztpraxen und dergleichen, in der Nahrungsmittelindustrie, dort insbesondere in der Viehhaltung, der weiterverarbeitenden Industrie, insbesondere Metzgereien, Bäckereien und dergleichen, und der Lagerhaltung. Anwendungen im privaten Sektor sind ebenfalls denkbar. Die vorzugebenden Grenzwerte können sich aus Erfahrungswerten im jeweiligen Sektor ergeben. Alternativ können sie sich auch durch gesetzliche Regelungen ergeben. Insbesondere in zu entkeimenden Bereichen, in welchen im bestimmungsgemäßen Fall der Nutzung Menschen arbeiten, ist der jeweilige erfindungsgemäße Grenzwert vorzugsweise ein gesetzlich vorgeschriebener Grenzwert, der sicherstellt, dass die Verwendung der einzelnen Komponenten im Desinfektionsmittel und damit das Desinfektionsmittel als solches für den Menschen keinerlei gesundheitsschädliche Wirkung entfalten. Vorzugsweise ist der jeweilige Grenzwert hierbei durch den gesetzlichen Arbeitsplatzgrenzwert (AGW; früher Maximale Arbeitsplatzkonzentration - MAK) für die Komponenten des Desinfektionsmittels gegeben. In Bereichen die mehreren gesetzlichen Regelungen unterworfen sind wird vorzugsweise der jeweils niedrigere Grenzwert eingehalten. Dies ist insbesondere in der Lebensmittelindustrie der Fall, wo sowohl Arbeitsplatzgrenzwerte als auch Grenzwerte der Lebensmittelhygiene-Verordnung (LMHV) gelten. Erfindungsgemäß wird die Gesamtkonzentration des Desinfektionsmittels im Aerosol so gewählt, dass die Einzelkonzentration sowohl des Oxidationsmittel als auch des Tensids unterhalb eines solchen Grenzwertes liegen. Bei aus dem Stand der Technik bekannten Verfahren zur Raumentkeimung wird typischerweise von Konzentrationen Gebrauch gemacht, die deutlich oberhalb der vorbeschriebenen Grenzwerte liegen, um eine Entkeimung überhaupt realisieren zu können. Die vorherrschende Meinung im Stand der Technik ist daher die, dass für eine ordnungsgemäße Entkeimung vergleichsweise hohe Konzentrationen an Wirkstoffen eingesetzt werden müssen, die für Menschen, Tiere, Lebensmittel und dergleichen schädlich sind. Die Erfindung schlägt hier in bewusster Abkehr vom Stand der Technik einen neuen Weg ein.

Der Anmelder hat überraschend herausgefunden, dass ein Desinfektionsmittel wenigstens bestehend aus einem Oxidationsmittel und einem Tensid in einer vorbeschriebenen Konzentration im Aerosol eine niemals für möglich gehaltene Wirksamkeit gegenüber Keimen aufweist. Vorzugsweise wird eine erfindungsgemäße Entkeimung in überraschender Weise bereits bei vergleichsweise geringeren Gesamtkonzentrationen erreicht. Erfindungsgemäß ist bereits eine Gesamtkonzentration von 0,01 ml bis 0,05 ml Desinfektionsmittel/m³ Raumluft für eine erfindungsgemäße Raumentkeimung ausreichend. Als besonders vorteilhaft hat sich hierbei eine Konzentration von 0,02 ml Desinfektionsmittel/m³ Raumluft erwiesen. Die verwendeten Gesamtkonzentrationen liegen damit deutlich unterhalb der AGW von für die Bildung des erfindungsgemäßen Desinfektionsmittels geeigneten Oxidationsmitteln wie insbesondere, Natriumhypochlorit (0,5 ml/m³), Wasserstoffperoxid (0,5 ml/m³), Ozon (0,1 ml/m³) oder Peroxyessigsäure (10 ml/m³). Gleichermaßen liegen die Gesamtkonzentrationen des Desinfektionsmittels im Aerosol deutlich unterhalb der für Stäube im Allgemeinen und für Tenside im speziellen geltenden AGW von ca. 10 ml/m³.

Das zur Entkeimung verwendete Aerosol ist damit nicht nur für Menschen gesundheitlich unbedenklich sondern darüber hinaus wirksam gegenüber Keimen und beeinträchtigt in keiner Weise die bestimmungsgemäße Nutzung des zu entkeimenden Raumes, wodurch das erfindungsgemäße Verfahren durch seine kontinuierliche Anwendung für eine initiale Entkeimung sorgt und darüber hinaus eine Wiederverkeimung in vorteilhafter Weise verhindert. Insbesondere im Gesundheitswesen kann das erfindungsgemäße Verfahren damit ohne Rücksichtnahme von im Raum anwesenden Menschen kontinuierlich durchgeführt werden. Eine Wiederverkeimung, wie sie bei den aus dem Stand der Technik bekannten Verfahren auftritt, kann mit der erfindungsgemäßen Ausgestaltung vorteilhafterweise vermieden werden, so dass in dem zu entkeimenden Raum auch bei Einbringung von frischen Keimen in besagten Raum, durch zum Beispiel Lüften, ein konstant niedriges Keimniveau beibehalten werden kann.

Gemäß der Erfindung wird aus dem Desinfektionsmittel ein schwebfähiges Aerosol erzeugt. Im Stand der Technik ist es demgegenüber aufgrund des Einsatzes von Düsen, insbesondere Hohlkegeldüsen bislang nicht möglich, ein schwebfähiges Aerosol zu erzeugen. Erfindungsgemäß wird das Desinfektionsmittel mit Luft gemischt. Luft dient mithin als Trägermedium für das Desinfektionsmittel. Das Desinfektionsmittel kann auf dem Wege des Luftkonvektion in einfacher Weise in dem zu entkeimenden Raum oder in auch nur in Teilen hiervon verteilt werden. Vorteilhafterweise wird hierdurch eine Entkeimung der Luft selbst und sämtlicher Oberflächen im zu entkeimenden Raum erreicht. Insbesondere Oberflächen, die im Stand der Technik in der Regel durch Wischen nur unzureichend entkeimt wurden, können durch die Erfindung nahezu vollständig entkeimt werden. Ferner ist es durch die Verwendung von Luft als Trägermedium nunmehr möglich auch Oberflächen zu entkeimen, die durch bloßes Wischen nicht entkeimt werden können. Dazu zählen insbesondere raue Oberflächen, poröse Oberflächen, texturierte Oberflächen, textile Oberflächen, wie insbesondere Vorhänge, Bettwäsche, Kleidung und dergleichen. Das Aerosol dringt in mikroskopische Strukturen dieser Oberflächen ein und entkeimt diese auch an für herkömmliche Verfahren unzugänglichen Stellen. Vorzugsweise wird Luft in Form von Umgebungsluft verwendet. Alternativ zu oder in Kombination mit der Umgebungsluft kann es vorgesehen sein, ein eigens hergestelltes luftähnliches Gasgemisch zu verwenden. Luftähnlich bedeutet in diesem Zusammenhang, dass das Gasgemisch Stickstoff und Sauerstoff in einem für den Metabolismus von Menschen und/oder Tieren ausreichenden Anteil enthält. Insbesondere Für den Fall, dass die Umgebungsluft nicht oder nicht als solches für die Erzeugung des Aerosols geeignet ist oder aus anderen Gründen nicht als Trägermedium verwendet werden kann, ist dies besonders vorteilhaft.

Schwebfähigkeit des Aerosols ist vorteilhaft, da hierdurch die Verteilbarkeit des Aerosols verbessert wird. Es ist insbesondere möglich, auch entfernte Raumbereiche mit Aerosol zu beaufschlagen, ohne dass das Desinfektionsmittel zuvor zu Boden sinkt. Bei der Erzeugung des Aerosols wird das Desinfektionsmittel gemäß der Erfindung daher zu feinen Tröpfchen mit einem Durchmesser unterhalb von 10 µm zerstäubt. Vorteilhafterweise kann auf diese Art ein schwebfähiges und stabiles Aerosol erzeugt werden. Es hat sich ferner herausgestellt, dass Tröpfchen mit einem solchen Durchmesser insbesondere bei strukturierten und/oder porösen Oberflächen, wie sie insbesondere bei Textilien vorkommen, aufgrund einer verbesserten Materialgängigkeit zu einer besonders effektiven Entkeimung führen. Zur weiteren Verbesserung der Materialgängigkeit ist erfindungsgemäß vorgesehen, Tröpfchen mit einem Durchmesser unterhalb von 5 µm, bevorzugt zwischen 1 µm und 3 µm zu erzeugen.

Erfindungsgemäß wird das Aerosol nach dessen Erzeugung kontinuierlich in den zu entkeimenden Raum eingebracht. Dies kann gemäß einer bevorzugten Ausgestaltung der Erfindung direkt und unmittelbar geschehen. Hierzu kann eine für die Durchführung des Verfahrens geeignete Vorrichtung in dem zu entkeimenden Raum bzw. dem zu entkeimenden Raumbereich aufgestellt werden. Vorzugsweise ist eine solche Vorrichtung mobil ausgebildet, um verschiedene Räume oder Raumbereiche je nach Wunsch oder Notwendigkeit zu entkeimen. Vorzugsweise weist die Vorrichtung hierzu Bewegungsmittel auf. Die Bewegungsmittel können bevorzugt als Rollen, Räder, Kufen oder dergleichen ausgebildet sein. Das erfindungsgemäße Verfahren ist vorzugsweise für jede Raumgröße skalierbar. Hierzu wird gemäß einer bevorzugten Ausführungsform der Erfindung die Leistungsfähigkeit der Vorrichtung an die Größe des zu entkeimenden Raumes angepasst. Gemäß einer weiteren, mit der vorgenannten Ausgestaltung kombinierbaren Ausgestaltung der Erfindung wird die Zahl der in einem Raum aufzustellenden Vorrichtungen zum Zwecke der Skalierbarkeit an die Größe des Raumes angepasst. Auf diesem Wege ist es möglich, das erfindungsgemäße Verfahren an jede beliebige Raumgröße anzupassen.

Gemäß einer alternativen Ausgestaltung der Erfindung wird das Aerosol mittelbar in den zu entkeimenden Raum eingebracht. Es ist hierbei nicht notwendig, die Vorrichtung in dem zu entkeimenden Raum aufzustellen. Vielmehr ist diese lediglich leitungstechnisch mit dem zu entkeimenden Raum verbunden. Eine solche Verfahrensführung erlaubt vorteilhafterweise eine räumliche Entkoppelung der Aerosolerzeugung von deren Verwendungsort. Hieraus ergibt sich eine ganze Reihe von Vorteilen. Einerseits sind etwaige mit der Aerosolherstellung einhergehende Effekte, wie insbesondere eine als unangenehm empfundene Geräuschentwicklung in dem zu entkeimenden Raum nicht wahrnehmbar. Dies ist insbesondere im Krankenhaus wo typischerweise ruhebedürftige Personen behandelt werden von Vorteil. Andererseits ist die Vorrichtung auch optisch nicht in dem zu entkeimenden Raum präsent, wodurch das Erscheinungsbild des Raumes nicht unangemessen gestört wird. Insbesondere in Arztpraxen, in denen regelmäßig viel Wert auf die Inneneinrichtung gelegt wird, ist eine solche Entkopplung von Vorteil. Vorzugsweise ist die Vorrichtung leitungstechnisch an mehr als nur einen Raum angeschlossen, wodurch mit einer Vorrichtung mehrere Räume entkeimt werden können. Bevorzugt ist die Anordnung der Vorrichtung in einem bereits bestehenden Belüftungssystem eines Gebäudes, welches eine Vielzahl von Räumen miteinander verbindet. Vorzugsweise wird die Vorrichtung an einem Knotenpunkt des Belüftungssystems angeordnet, um möglichste viele Räume gleichermaßen zu entkeimen. Der in einem solchen Belüftungssystem vorliegende Luftstrom umspült dabei die Vorrichtung. Er dient hierbei vorteilhafterweise als Trägermedium für das Desinfektionsmittel im Aerosol.

Die Erfindung betrifft darüber hinaus ein Desinfektionsmittel zum Einsatz im erfindungsgemäßen Verfahren. Dieses zeichnet sich durch eine besondere Wirkstoffkombination aus wenigstens einem Oxidationsmittel und einem Tensid aus.

Der Anmelder hat herausgefunden, dass die erfindungsgemäße Wirkstoffkombination bei der Keimbekämpfung eine synergetische Wirkung entfaltet und somit besonders effektiv ist. Es ist hierdurch möglich, vergleichsweise geringe Konzentrationen an Desinfektionsmittel zur Entkeimung zu verwenden, was eine für den Menschen unschädliche, kontinuierliche Verfahrensführung überhaupt erst ermöglicht.

Ohne auf diesen Mechanismus beschränkt zu sein, wird anmelderseitig vermutet, dass das Oxidationsmittel die äußeren Strukturen des jeweiligen Keims angreift und dem Tensid auf diese Art den Zugang zum Inneren des Keimes ermöglicht, wo dieser seine für den Keim schädliche Wirkung mit vergleichsweise hoher Wirksamkeit entfalten kann.

Gemäß der Erfindung ist das Oxidationsmittel aus Wasserstoffperoxid (H₂O₂) gebildet. Es zeichnet sich in vorteilhafter Weise durch ein vergleichsweise hohes Standardelektrodenpotential (Redoxpotential unter Standardbedingungen) von 1,78 V aus, womit es zu den starken Oxidationsmitteln gehört. Neben seiner Rolle als Oxidationsmittel ist Wasserstoffperoxid im Bereich der Raumentkeimung besonders vorteilhaft, da es während der Entkeimung zu Wasser (H₂O) reduziert wird, so dass keinerlei schädliche Nebenprodukte entstehen. Ferner neigt Wasserstoffperoxid dazu im Rahmen einer Disproportionierungsreaktion in Wasser und Sauerstoff zu zerfallen. Im Bereich der Raumentkeimung hat sich dieses Verhalten neben der eigentlichen Entkeimung als vorteilhaft erwiesen, da eine sukzessive Anreicherung der Umgebungsluft mit Sauerstoff stattfindet, was von den sich im Raum aufhaltenden Personen im Allgemeinen als angenehm und "frisch" empfunden wird. Hieraus ergibt sich insbesondere mit der Wirksamkeit des Desinfektionsmittels gegenüber Feinstaub mit Bezug auf die Luftauffrischung ein besonders positiver synergetischer Effekt.

Das erfindungsgemäß als Komponente im Desinfektionsmittel eingesetzte Tensid ist gemäß einem bevorzugten Merkmal der Erfindung ein pflanzliches Tensid. Pflanzlich bedeutet in diesem Zusammenhang, dass das Tensid aus pflanzlichen Rohstoffen, insbesondere Fetten gewinnbar ist. Es kann vorzugsweise vorgesehen sein, dass das Desinfektionsmittel mehr als ein Tensid enthält, um eine möglichst breite Entkeimungswirksamkeit zu erreichen. Es hat sich diesbezüglich herausgestellt, dass unterschiedliche Tenside unterschiedlich wirksam gegenüber verschiedenen Arten von Keimen sind. Vorzugsweise ist es hierbei vorgesehen, dass sämtliche im Desinfektionsmittel eingesetzten Tenside aus einer einzigen pflanzlichen Quelle gewinnbar sind. Dies kann vorliegend insbesondere Kokosöl sein. Der Anmelder hat herausgefunden, dass die aus den im Kokosöl enthaltenen Fettsäuren gewinnbaren Tenside, insbesondere die Carboxylate der Palmitin-, Laurin-, Myristin- und Ölsäure, eine besonders hohe Wirksamkeit und ein breites Spektrum bei der Keimbekämpfung aufweisen.

Es hat sich bei Wirksamkeitstests des Desinfektionsmittels gezeigt, dass die Wirksamkeit des Desinfektionsmittels von der Art und der genauen quantitativen Zusammensetzung der Einzelkomponenten des Desinfektionsmittels abhängig ist. Es ist prinzipiell möglich, das erfindungsgemäße Aerosol sowohl aus einem Desinfektionsmittel im festen, als auch im flüssigen Zustand zu erzeugen. Es ist jedoch erfindungsgemäß vorgesehen, das Desinfektionsmittel aufgrund der einfacheren Handhabung als wässrige Lösung auszubilden. Vorzugsweise wird hierzu ausschließlich vollentsalztes Wasser verwendet, um ein möglichst reines Desinfektionsmittel bereitzustellen, und auf diesem Wege mögliche Nebenwirkungen oder Nebenreaktionen mit den Wirkstoffen zu vermeiden. Erfindungsgemäß besteht das Desinfektionsmittel wenigstens aus 95 Gew.% bis 98 Gew.% vollentsalztem Wasser, 1,5 Gew.% bis 4,9 Gew.% Wasserstoffperoxid und 0,1 Gew.% bis 0,5 Gew.% wenigstens eines Tensids. Gemäß einer besonders vorteilhaften Ausgestaltung der Erfindung besteht das Desinfektionsmittel wenigstens aus 97 Gew.% vollentsalztem Wasser, 2,7 Gew.% Wasserstoffperoxid und 0,3 Gew.% eines aus Kokosöl gewinnbaren Tensidgemisches. Vorgenannte Zusammensetzungen haben sich hinsichtlich der Entkeimungszeit, der Entkeimungswirksamkeit sowie des Entkeimungsspektrums als besonders vorteilhaft erwiesen.

Gemäß einer besonders bevorzugten Ausgestaltung der Erfindung ist es vorgesehen, dem Desinfektionsmittel Ozon als weiteres Oxidationsmittel beizufügen. Verfahrensseitig kann dies entweder nachträglich im Laufe des Entkeimungsverfahrens oder bereits im Vorfeld des Verfahrens durch Bereitstellung eines Ozon enthaltenden Desinfektionsmittels realisiert werden. In der elektrochemischen Spannungsreihe liegt das Ozon mit einem Standardelektrodenpotential von 2,075 V über dem von Wasserstoffperoxid und ist dem entsprechend ein stärkeres Oxidationsmittel. Die in das Desinfektionsmittel eingebrachte Konzentration an Ozon ist im Sinne der erfindungsgemäßen Durchführung des Verfahrens ebenfalls so gewählt, dass diese im Aerosol für Menschen nicht gesundheitsschädlich ist. Es hat sich herausgestellt, dass eine solche Konzentration an Ozon in Verbindung mit dem im Desinfektionsmittel enthaltenen Wasserstoffperoxid das Gesamtredoxpotential auf bis zu 2,86 V erhöht. Es ist durch die vorgenannte Kombination von Oxidationsmitteln möglich, das Redoxpotential über den Wert ihrer Einzelkomponenten zu steigern, so dass das Gesamtpotential in synergetischer Weise das Standardelektrodenpotential von Fluor erreicht.

Die Erfindung betrifft ferner eine Vorrichtung zur Durchführung des erfindungsgemäßen Verfahrens. Erfindungsgemäß verfügt die Vorrichtung über einen Desinfektionsmitteltank zur Bereitstellung eines Desinfektionsmittels. Der Desinfektionsmitteltank ist vorzugsweise mit einem Vorratsbehälter leitungstechnisch verbunden. Der Vorratsbehälter dient der Auffüllung des Desinfektionsmitteltanks. Das Fassungsvermögen des Vorratsbehälters ist an die Größe der Vorrichtung anpassbar. Typischerweise nimmt dieser je nach Größe des Geräts zwischen 10 I und 20 I Desinfektionsmittel auf. Es sind jedoch in Abhängigkeit der/des zu entkeimenden Räume/Raumes auch wesentlich größere Behälter denkbar. Vorzugsweise sind Vorratsbehälter und Desinfektionsmitteltank unter Zwischenschaltung einer Pumpe miteinander verbunden. Die Pumpe kann vorzugsweise als Schlauchpumpe, Membranpumpe oder dergleichen ausgebildet sein. Mittels der Pumpe wird das Desinfektionsmittel vorzugsweise aus dem Vorratsbehälter in den Desinfektionsmitteltank gepumpt. Dies kann vorzugsweise automatisch geschehen. Zu diesem Zweck verfügt der Desinfektionsmitteltank über einen Füllstandssensor. Für die bestimmungsgemäße Verfahrensführung ist es vorrichtungsseitig bevorzugt, dass im Desinfektionsmitteltank eine bestimmte, vorgebbare Füllstandhöhe eingehalten wird. Um diese Füllstandhöhe innerhalb des Desinfektionsmitteltanks einzuhalten, ist vorzugsweise der Füllstandssensor vorgesehen. Dieser ist steuerungstechnisch mit der Pumpe verbunden, so dass aus dem Vorratsbehälter bei Unterschreiten der Füllstandhöhe automatisch Desinfektionsmittel nachgepumpt wird, um den zur Funktionstüchtigkeit insbesondere der Zerstäubungsmembran notwendigen Flüssigkeitsspiegel innerhalb des Desinfektionsmitteltanks einzuhalten.

Die Vorrichtung weist erfindungsgemäß ferner eine mit dem Desinfektionsmitteltank leitungstechnisch verbundene Aerosolerzeugungseinheit zur Erzeugung eines Aerosols aus dem Desinfektionsmittel auf. Gemäß einem bevorzugten Merkmal der Erfindung verfügt die Aerosolerzeugungseinheit über eine Zerstäubungsmembran. Diese ist vorzugsweise derart ausgebildet, dass sie in einer einstellbaren Frequenz oszillieren kann. Vorzugsweise wird die Frequenz in Abhängigkeit der zu erzeugenden Aerosolmenge eingestellt. Ferner bevorzugt kann die Frequenz in Abhängigkeit der Größe der zu erzeugenden Desinfektionsmitteltröpfchen eingestellt werden. Vorzugsweise ist es vorgesehen, die Zerstäubungsmembran mit einer Frequenz im Ultraschallbereich oszillieren zu lassen. Hierdurch können besonders kleine Desinfektionsmitteltröpfchen im Bereich unterhalb von 10 µm erzeugt werden.

Die Vorrichtung weist erfindungsgemäß darüber hinaus eine mit der Aerosolerzeugungseinheit leitungstechnisch verbundenen Ventilationseinheit zum Ansaugen der Umgebungsluft und zum Ausleiten des Aerosols auf. Mittels dieser wird die Raumluft, vorzugsweise die Umgebungsluft, angesaugt und an der Aerosolerzeugungseinheit vorbeibewegt, so dass von der Aerosolerzeugungseinheit erzeugte Desinfektionsmitteltröpfchen in den Luftstrom eingebracht und Alsdann zurück in den zu entkeimenden Raum verbracht werden können.

Wie bereits beschrieben, kann dem Desinfektionsmittel während des kontinuierlichen Betriebs Ozon beigemischt werden. Hierzu verfügt die Vorrichtung über eine Ozonerzeugungseinheit. Die Ozonerzeugungseinheit kann vorzugsweise in Form von Ozonkerzen ausgebildet sein. Vorzugsweise sind die Ozonkerzen mit dem Desinfektionsmitteltank zur Einleitung des erzeugten Ozons in das Desinfektionsmittel leitungstechnisch verbunden ist. Neben Ozonkerzen können auch weitere aus dem Stand der Technik bekannte Ozonerzeugungseinheiten verwendet werden. Diese können entweder alleine oder in Kombination miteinander betrieben werden.

Vorzugsweise verfügt die Vorrichtung über eine Fehlerausgabeeinheit. Diese dient der Anzeige von mit dem Betrieb der Vorrichtung insgesamt oder der Einzelkomponenten in Verbindung stehenden Fehlern. Insbesondere können dies Fehler hinsichtlich des Füllstandes im Desinfektionsmitteltank sein. Dieser kann durch einen Defekt der Pumpe, des Füllstandssensors oder ähnlichem verursacht werden. Ferner können Fehler betreffend die Aerosolerzeugungseinheit angezeigt werden. Insbesondere kann ein Membrandefekt angezeigt werden. Auch Fehler im Zusammenhang mit der Ventilationseinheit, insbesondere ein defekter Antrieb oder dergleichen, können angezeigt werden. Insgesamt führt dies zu einer Vereinfachung einer Wartung und gegebenenfalls einer Reparatur der Vorrichtung, da Fehlerquellen erkannt und direkt angezeigt werden können. Vorzugsweise ist die Fehlerausgabeeinheit als LED-Feld ausgebildet. Vorzugsweise ist es vorgesehen, jedem Bauteil eine andersfarbige LED zuzuordnen. Besonders bevorzugt werden Fehler des Desinfektionsmitteltanks mittels einer grünen LED angezeigt. Ferner bevorzugt werden Fehler der Aerosolerzeugungseinheit mittels einer roten LED angezeigt. Ebenfalls bevorzugt werden Fehler der Ventilationseinheit mittels einer gelben LED angezeigt.

Gemäß einer besonders bevorzugten Ausgestaltung der Erfindung ist die Fehlerausgabeeinheit als Displayeinheit ausgebildet. Vorzugsweise handelt es sich um ein Touchdisplay, um unterschiedliche Fehlerstände abzurufen anzuzeigen. Zu diesem Zweck verfügt die Vorrichtung über einen Datenspeicher, in welchem Fehlermeldungen abrufbar abgespeichert werden.

Gemäß einer bevorzugten Ausgestaltung der Erfindung verfügt die Vorrichtung ferner über eine Steuereinrichtung. Diese dient insbesondere der Steuerung der Einzelkomponenten. Insbesondere können damit Flüssigkeitsströme zwischen Vorratsbehälter und Desinfektionsmitteltank gesteuert werden. Ferner ist der Flüssigkeitsstrom vom Desinfektionsmitteltank zur Aerosolerzeugungseinheit durch die Steuereinrichtung steuerbar. Zu diesem Zweck kann die Steuereinrichtung mit Pumpen und/oder Ventilen und/oder sonstigen strömungsregelnden Einrichtungen steuerungstechnisch verbunden sein. Die Bereitstellung einer Steuerungseinrichtung erlaubt in vorteilhafter Weise einen automatisierten Betrieb der erfindungsgemäßen Vorrichtung. Hierdurch können insbesondere der Wartungsaufwand verringert und die Effizienz des Verfahrens insgesamt verbessert werden. Gemäß einer weiteren bevorzugten Ausgestaltung der Erfindung kann die Steuereinrichtung zu Detektion von Fehlerzuständen ausgebildet sein. Zu diesem Zweck ist die Steuerungseinrichtung mit entsprechenden Sensoren verbunden. Diese Sensoren können hierzu bevorzugt in den Einzelkomponenten angeordnet sein. Ferner ist die Steuerungseinrichtung hierzu mit der Fehlerausgabeeinheit verbunden. Vorher detektierte Fehlerzustände können hierdurch an die Fehlerausgabeeinheit gemeldet werden, die diese in einem solchen Fall anzeigt.

Ferner betrifft die Erfindung ein System zur Entkeimung von Räumen oder Bereichen hiervon.

Das System besteht aus einer erfindungsgemäßen Vorrichtung und einem erfindungsgemäßen Desinfektionsmittel. Durch das erfindungsgemäße System wird eine dauerhaft sichere Entkeimung erreicht und eine Wiederverkeimung wirksam vermieden. Die aus dem Stand der Technik unbekannte synergetische Kombination aus erfindungsgemäßer Vorrichtung und erfindungsgemäßem Desinfektionsmittel ermöglichen die kontinuierliche Verfahrensführung des erfindungsgemäßen Verfahrens. Die bevorzugten Merkmale der Einzelkomponenten des erfindungsgemäßen Systems wirken in synergetischer Weise für eine insgesamte weitere Verbesserung des erfindungsgemäßen Systems über die Summe ihrer Einzelteile hinaus.

Die Erfindung wird nachfolgend anhand eines für den Fachmann nicht beschränkend zu verstehenden Ausführungsbeispiels näher erläutert. Dabei zeigt:

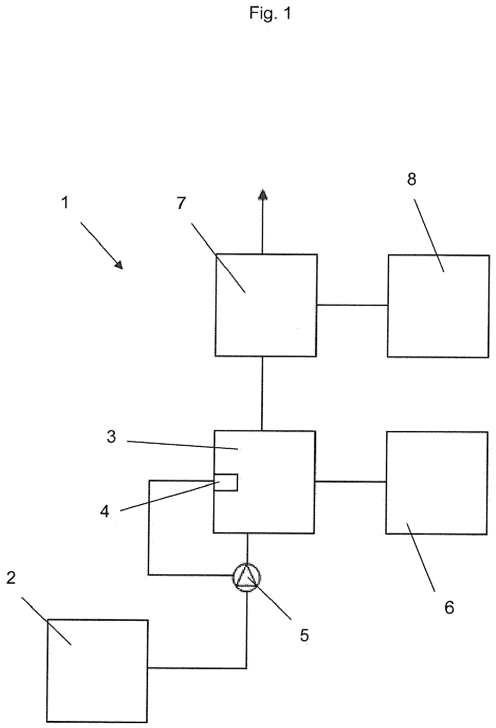

- Fig. 1: eine erfindungsgemäße Vorrichtung zur Durchführung des erfindungsgemäßen Verfahrens in schematischer Darstellung.

Fig. 1 zeigt eine erfindungsgemäße Vorrichtung 1 mit einem Vorratsbehälter 2. Der Vorratsbehälter 2 ist leitungstechnisch mit dem Desinfektionsmitteltank 3 verbunden. Die Verbindung des Desinfektionsmitteltanks 3 und des Vorratsbehälters 2 ist im vorliegenden Beispiel unter Zwischenschaltung einer Schlauchpumpe 5 realisiert. Der Desinfektionsmitteltank 3 verfügt vorliegend über einen Füllstandssensor 4. Der Füllstandssensor 4 ist steuerungstechnisch mit der Schlauchpumpe 5 verbunden. Auf diesem Wege wird bei Unterschreiten eines vorgebbaren Füllstandes im Desinfektionsmitteltank 3 automatisch Desinfektionsmittel aus dem Vorratsbehälter 2 in den Desinfektionsmitteltank 3 verbracht. Hierdurch wird vorteilhafterweise ein kontinuierlicher Betrieb ermöglicht.

Das Desinfektionsmittel ist in diesem Ausführungsbeispiel als wässrige Lösung ausgebildet. Sie besteht demgemäß aus 97 Gew.% vollentsalztem Wasser, 2,7 Gew.% Wasserstoffperoxid und 0,3 Gew.% Tensiden auf Kokosölbasis. Eine solche Wirkstoffkombination hat sich hinsichtlich der Wirksamkeit gegenüber üblichen Keimen als besonders wirkungsvoll erwiesen, so dass vergleichsweise geringe Konzentrationen zu einer im Wesentlichen vollständigen Entkeimung führen. Darüber hinaus weist ist vorgenannte Desinfektionslösung ein vergleichsweise breites Keimbekämpfungsspektrum auf. Es hat sich herausgestellt, dass es gegen Bakterien, Viren, Sporen und Pilze gleichermaßen wirksam ist. Ferner findet durch die Verwendung von Wasserstoffperoxid eine durch die Bildung von Sauerstoff bedingte Luftauffrischung statt, die von anwesenden Personen als angenehm empfunden wird.

Vorliegend ist der Desinfektionsmitteltank 3 mit einer Ozonerzeugungseinheit 6 leitungstechnisch verbunden. Die Ozonerzeugungseinheit 6 ist vorliegend in Form einer Ozonkerze ausgebildet. Das in dem mittels der Ozonkerze erzeugte Ozon wird in die Desinfektionsmittellösung, eingeleitet. Hierdurch löst sich das gebildete Ozon wenigstens teilweise im Desinfektionsmittel. Das derart modifizierte Desinfektionsmittel wird der Aerosolerzeugungseinheit 7 zugeführt. Die Aerosolerzeugungseinheit 7 verfügt vorliegend über eine Zerstäubungsmembran (nicht gezeigt). Die Zerstäubungsmembran oszilliert mit einer Frequenz im Ultraschallbereich. Hierdurch können Desinfektionsmitteltröpfchen mit einem Durchmesser von 3 µm erzeugt werden. Es hat sich herausgestellt, dass eine solche Tröpfchengröße hinsichtlich der Schwebfähigkeit des Aerosols besonders vorteilhaft ist. Ein derartiges Aerosol erreicht auch Bereiche des zu entkeimenden Raumes, welche vergleichsweise weit von der Vorrichtung 1 entfernt sind, ohne zuvor abzusinken. Durch ein solches Aerosol können ferner raue, poröse, insbesondere textile Oberflächen, welche herkömmlichen Desinfektionsverfahren nicht zugänglich sind, besonders wirkungsvoll entkeimt werden. Dies ist insbesondere der in der geringen Tröpfchengröße liegenden, vorteilhaften Materialgängigkeit der Aerosoltröpfchen geschuldet.

Die Aerosolerzeugungseinheit 7 ist mit einer Ventilationseinheit 8 leitungstechnisch verbunden. Die Ventilationseinheit 8 saugt eine die Vorrichtung umgebende Raumluft zum Zwecke der Aerosolerzeugung an. Die Raumluft wird an der Zerstäubungsmembran vorbeibewegt, und dient somit als Trägermedium für das Desinfektionsmittel. Die an der Zerstäubungsmembran erzeugten Mikrotröpfchen werden zu diesem Zweck in den Luftstrom eingebracht. Alsdann wird das erzeugte Aerosol aus der Vorrichtung ausgeleitet. Die Ausleitung kann erfindungsgemäß entweder unmittelbar in den zu entkeimenden Raum erfolgen. Hierzu kann die Vorrichtung 1 in dem zu entkeimenden Raum bzw. dem zu entkeimenden Raumbereich aufgestellt werden. In diesem Fall ist die Vorrichtung 1 mobil ausgebildet, um verschiedene Räume oder Raumbereiche je nach Wunsch oder Notwendigkeit zu entkeimen. Zu diesem Zweck weist die Vorrichtung 1 Rollen auf.

Alternativ kann das Aerosol mittelbar in den zu entkeimenden Raum eingebracht werden. Hierzu ist die Vorrichtung 1 in einem bereits bestehenden Belüftungssystem eines Gebäudes, welches eine Vielzahl von Räumen miteinander verbindet angeordnet, um möglichste viele Räume gleichermaßen zu entkeimen. Der in einem solchen Belüftungssystem vorliegende Luftstrom umspült dabei die Vorrichtung. Er dient hierbei vorteilhafterweise als Trägermedium für das Desinfektionsmittel im Aerosol.

Vorliegend verfügt die Vorrichtung über eine nicht dargestellte Fehlerausgabeeinheit. Die Fehlerausgabeeinheit ist vorliegend als LED-Feld ausgebildet. Fehler des Desinfektionsmitteltanks werden mittels einer grünen LED angezeigt. Fehler der Aerosolerzeugungseinheit werden mittels einer roten LED angezeigt. Fehler der Ventilationseinheit werden mittels einer gelben LED angezeigt. Vorteilhafterweise führt dies insgesamt zu einer Vereinfachung der Wartung und gegebenenfalls der Reparatur der Vorrichtung 1, da Fehlerquellen erkannt und direkt angezeigt werden können.

### Bezugszeichenliste

- 1: Entkeimungsvorrichtung
- 2: Vorratsbehälter
- 3: Desinfiektionsmitteltank
- 4: Flüssigkeitsmesser
- 5: Schlauchpumpe
- 6: Ozonkerze
- 7: Aerosolerzeugungseinheit
- 8: Ventilationseinheit

## Patentansprüche

1. Verfahren zur Entkeimung von Räumen oder Bereichen hiervon, bei dem ein Desinfektionsmittel verwendet wird, welches ein Oxidationsmittel und ein Tensid aufweist, bei dem aus dem Desinfektionsmittel ein schwebfähiges Aerosol erzeugt und kontinuierlich in den zu entkeimenden Raum eingebracht wird, bei dem die Gesamtkonzentration des Desinfektionsmittels im Aerosol eingestellt wird, wobei jeweils ein maximaler Grenzwert für die Einzelkonzentration des Oxidationsmittels und des Tensids im Aerosol vorgegeben wird, **dadurch gekennzeichnet, dass** das Aerosol aus einem Desinfektionsmittel wenigstens bestehend aus 95 bis 98 Gew.-% vollentsalztem Wasser, 1,5 bis 4,9 Gew.-% H₂O₂ und 0,1 bis 0,5 Gew.-% wenigstens eines Tensids, erzeugt wird, wobei zwischen 0,01 und 0,05 ml Desinfektionsmittel pro 1 m³ Raumluft mittels einer mit einer Ultraschallfrequenz oszillierenden Membran zu Tröpfchen mit einem Durchmesser unterhalb von 5 µm zerstäubt werden.

2. Verfahren nach Anspruch 1, **dadurch gekennzeichnet, dass** vor der Erzeugung des Aerosols Ozon erzeugt und in das Desinfektionsmittel eingeleitet wird.

## Claims

1. A method for the sterilization of rooms or parts thereof, in which a disinfactant is used which comprises an oxidizing agent and a surfactant, in which a floatable aerosol is generated from the disinfectant and continuously introduced into the room to be sterilized, in which the total concentration of the disinfectant in the aerosol is set, wherein respectively a maximum limit value of the individual concentrations of the oxidizing agent and the surfactant in the aerosol is predetermined, **characterized in that** the aerosol is generated from a disinfectant which at least consists of 95 to 98 % by weight completely desalinated water, 1.5 to 4.9 % by weight H₂O₂ and 0.1 to 0.5 % by weight of at least one surfactant, wherein between 0.01 and 0.05 ml disinfectant per 1 m³ room air will be atomized to droplets having a diameter of less than 5 µm by means of a membrane which oscillates with an ultrasound frequency.

2. A method according to claim 1, **characterized in that** ozone is generated and introduced into the disinfectant before the aerosol is generated.

## Revendications

1. Procédé de stérilisation des pièces ou des parties de celles-ci, dans lequel on utilise un produit désinfectant, qui comprend un agent oxydant et un tensioactif, dans lequel un aérosol flottant est généré à partir du produit désinfectant et introduit de manière continue dans la pièce à stériliser, dans lequel la concentration totale du produit désinfectant dans l'aérosol est réglée, une valeur limite maximale de la concentration individuelle de l'agent oxydant et du tensioactif dans l'aérosol étant respectivement prédéterminée, **caractérisé en ce que** l'aérosol est généré à partir d'un produit désinfectant qui consiste au moins en 95 à 98 % en poids de l'eau complètement déminéralisée, en 1,5 à 4,9 % en poids de H₂O₂ et en 0,1 à 0,5 % en poids d'au moins un tensioactif, entre 0,01 et 0,05 ml du produit désinfectant par 1 m³ d'air ambiant étant atomisés pour former des gouttelettes ayant un diamètre de moins de 5 µm par moyen d'une membrane oscillante avec une fréquence ultrasonique.

2. Procédé selon la revendication 1, **caractérisé en ce que** de l'ozone est généré et introduit dans le produit désinfectant avant de générer l'aérosol.
